Europäisches Patentamt

European Patent Office

Office européen des brevets

(19)

(11) EP 0 925 761 A1

(12) **EUROPÄISCHE PATENTANMELDUNG**

(43) Veröffentlichungstag:
30.06.1999 Patentblatt 1999/26

(51) Int. Cl.$^6$: **A61B 17/39**

(21) Anmeldenummer: 98120431.6

(22) Anmeldetag: 28.10.1998

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 23.12.1997 DE 19757720

(71) Anmelder: Sulzer Osypka GmbH
79639 Grenzach-Wyhlen (DE)

(72) Erfinder:
 • Geistert, Wolfgang, Dr.
  79618 Rheinfelden (DE)
 • Uphoff, Peter, Dr.
  79211 Denzlingen (DE)

(74) Vertreter:
  Pellkofer, Dieter Dr. et al
  Manitz, Finsterwald & Partner GbR,
  Patent- und Rechtsanwälte,
  Robert-Koch-Strasse 1
  80538 München (DE)

(54) **Verfahren zum Betrieb einer Hochfrequenz-Ablationsvorrichtung und Vorrichtung für die Hochfrequenz-Gewebe-Ablation**

(57) Es wird ein Verfahren zum Betrieb einer Hochfrequenz-Ablationsvorrichtung mit einer mehrere regelbare Ausgänge aufweisenden HF-Energiequelle beschrieben, an deren Ausgänge eine Vielzahl von Elektroden anschließbar sind. Es wird für jeden Ausgang jeweils ein für die von der HF-Energiequelle abgegebene Leistung und/oder den Strom repräsentativer Leistungs- und/oder Stromwert erfaßt und die von der HF-Energiequelle abgegebene Spannung in Abhängigkeit von dem erfaßten Leistungs- und/oder Stromwert so geregelt, daß diese im wesentlichen einem vorgegebenen Leistungs- und/oder Stromwert entspricht. Zwischen den Strömen oder zwischen den Spannungen an den Ausgängen der HF-Energiequelle wird dabei jeweils eine vorgegebene Phasenbeziehung eingehalten. Weiterhin wird eine entsprechende Vorrichtung beschrieben.

Fig. 1

EP 0 925 761 A1

**Beschreibung**

Die vorliegende Erfindung betrifft ein Verfahren zum Betrieb einer Hochfrequenz-Ablationsvorrichtung, mit einer mehrere regelbare Ausgänge aufweisenden HF-Energiequelle, an deren Ausgänge eine Vielzahl von Elektroden anschließbar sind. Weiterhin ist die Erfindung auf eine Vorrichtung für die Hochfrequenz-Gewebe-Ablation gerichtet.

Ablationsvorrichtungen dieser Art werden beispielsweise zur Behandlung von Herzrhythmusstörungen verwendet. Dazu wird ein Ablationskatheter mit einer oder mehreren Elektroden an einer HF-Energiequelle angeschlossen und beispielsweise über eine Ader in das Innere des Herzens eingeführt. Die an dem Katheterende vorgesehenen Elektroden werden an die gewünschte Stelle an der Innen- oder Außenfläche des Herzens positioniert, woraufhin durch Zufuhr von hochfrequenter Energie die mit den Elektroden in Kontakt stehenden Bereiche thermisch verödet werden.

Durch die auf diese Weise erzeugten Läsionen werden die elektrischen Eigenschaften des behandelten Herzgewebes so verändert, daß vorhandene Herzrhythmusstörungen beseitigt werden.

Für bestimmte Arten von Herzrhythmusstörungen, für die größere Gewebegebiete verödet werden müssen, sind Katheter mit nur einer Elektrode schlecht geeignet, da die dabei erforderliche Aneinanderreihung einer Vielzahl von punktförmigen Läsionen bei schlagendem Herzen meist nur ungenügend und mit großem Zeitaufwand möglich ist.

Für die Verödung größerer Gewebegebiete können Katheter mit mehreren Elektroden verwendet werden. Dabei ist es vorteilhaft, wenn die Energieabgabe für jede Katheterelektrode individuell einstellbar ist, damit beispielsweise trotz unterschiedlicher Kühlungsverhältnisse an den unterschiedlichen Elektroden sowie trotz unterschiedlicher Lastwiderstände für jede Katheterelektrode die optimale, in dem Gewebe wirkende Temperatur einstellbar ist.

Üblicherweise wirken die einzelnen Katheterelektroden mit einer großflächig am Körper der zu behandelnden Person anliegenden, sogenannten indifferenten Elektrode zusammen, so daß bei einer Energiezufuhr an die Katheterelektroden jeweils Strom von den Katheterelektroden durch den Körper des Patienten zu der indifferenten Elektrode fließt. Da aufgrund der geringen Fläche der Katheterelektroden die Stromdichte unmittelbar am Übergang zwischen den Katheterelektroden und dem zu behandelnden Gewebe am höchsten ist, ist bei ausreichender Energiezufuhr die Temperatur in diesen Bereichen so hoch, daß die gewünschten Läsionen erzeugt werden.

Bei der Verwendung von Kathetern mit mehreren Elektroden tritt dabei folgendes Problem auf: Da je nach Bedarf an unterschiedlichen Katheterelektroden unterschiedliche Temperaturen oder zur Erreichung einer bestimmten Temperatur unterschiedliche Energien erforderlich sein können und darüber hinaus die an den Elektroden wirksam werdenden Lastwiderstände unterschiedlich sein können, muß die Energieabgabe über die einzelnen Katheterelektroden individuell eingestellt werden. Dies führt dazu, daß an den unterschiedlichen Katheterelektroden unterschiedliche Spannungs- und Stromwerte erforderlich sind. Insbesondere wenn die Katheterausgänge niedrige Ausgangswiderstände besitzen, treten zwischen Elektroden, an denen unterschiedliche Spannungswerte anliegen oder bei Phasenverschiebungen zwischen den Ausgangsspannungen, Ausgleichsströme auf. Bei diesen Ausgleichsströmen handelt es sich um Ströme, die von einer Katheterelektrode statt zu der indifferenten Elektrode, zu einer anderen Katheterelektrode fließen, an der eine andere Spannung anliegt als an der Elektrode, aus der der Strom austritt. Besteht zwischen den beiden Katheterelektroden eine hohe Potentialdifferenz, so kann der in die andere Elektrode einströmende Ausgleichsstrom zu unerwünscht hohen Stromdichten an dieser Elektrode führen, die wiederum unerwünschte Koagulationen an dieser Elektrode bewirken. Dabei macht sich dieser Effekt gerade an Elektroden besonders bemerkbar, an denen eigentlich keine oder nur eine geringe Energieabgabe gewünscht ist.

Aufgabe der Erfindung ist es, eine Vorrichtung und ein Verfahren der eingangs genannten Art so auszubilden, daß Ausgleichsströme zwischen den Ausgängen bzw. zwischen an den Ausgängen angeschlossenen Elektroden weitestgehend vermieden werden. Weiterhin soll eine kontinuierliche Energieabgabe möglich sein und die Vorrichtung einen hohen Wirkungsgrad aufweisen sowie der Schaltungsaufwand möglichst gering sein.

Ausgehend von einem Verfahren der eingangs genannten Art wird der das Verfahren betreffende Teil der Aufgabe dadurch gelöst, daß für jeden Ausgang jeweils ein für die von der HF-Energiequelle abgegebene Leistung und/oder den Strom repräsentativer Leistungs- und/oder Stromwert erfaßt wird, daß die von der HF-Energiequelle abgegebene Spannung in Abhängigkeit von dem erfaßten Leistungs- und/oder Stromwert so geregelt wird, daß dieser im wesentlichen einem vorgegebenen Leistungs- und/oder Stromwert entspricht und daß zwischen den Strömen oder zwischen den Spannungen an den Ausgängen der HF-Energiequelle jeweils eine vorgegebene Phasenbeziehung eingehalten wird.

Der die Vorrichtung betreffende Teil der Aufgabe wird erfindungsgemäß gelöst durch eine Vorrichtung der eingangs genannten Art mit zumindest einem Meßelement zur Erfassung von für die von der HF-Energiequelle am dem Ausgängen jeweils abgegebene Leistung und/oder den Strom repräsentativen Leistungs- und/oder Stromwerten und zumindest einem mit dem Meßelement verbundenen Regelelement zur Regelung der von der HF-Energiequelle abgegebenen Spannung in Abhängigkeit von dem an einem Istwerteingang des Regelelements anliegenden erfaßten Leistungs- und/oder Stromwert und einem an einem Sollwerteingang des Regelelements anliegenden vorgegebenen Leistungs- und/oder Stromwert, wobei die Ströme oder Spannungen an den Ausgängen der HF-Energiequelle jeweils eine vorgegebene Phasenbeziehung zueinander besitzen.

Die erfindungsgemäß ausgebildete Vorrichtung ist somit so ausgelegt, daß ihre Ausgänge das Verhalten einer Strom- oder Leistungsquelle besitzen. Dadurch werden Ausgleichsströme, die beispielsweise von einer Elektrode mit hoher Spannung zu einer Elektrode mit geringerer Spannung fließen und eine Rückspeisung in diese Elektrode bewirken würden, vermieden. Diese Rückspeisung würde dazu führen, daß durch den Ausgleichsstrom im Extremfall ein betragsmäßig unerwünscht hoher Strom an dieser Elektrode fließt, der eine unerwünschte Koagulation an dieser Elektrode bewirkt. Beispielsweise kann sich unter Umständen der Ausgleichsstrom von dem eingeprägten Ausgangsstrom der betroffenen Elektrode subtrahieren, was dazu führen kann, daß sich die Wirkrichtung des durch die betroffene Elektrode fließenden Stroms umkehrt.

Durch die erfindungsgemäße Strom- bzw. Leistungsregelung wird dieser Sollwertabweichung unmittelbar durch Nachregelung, beispielsweise durch eine Erhöhung der Ausgangsspannung entgegengewirkt, so daß wieder der gewünschte Strom durch die Elektrode fließt.

Nach einer bevorzugten Ausführungsform der Erfindung umfaßt die HF-Energiequelle zumindest eine regelbare Gleichspannungsquelle, die beispielsweise aus einer ungeregelten Gleichspannungsquelle und einem Spannungsregler besteht, und zumindest eine mit der Gleichspannungsquelle verbundene Schaltstufe, wobei zur Erzeugung der HF-Ausgangsspannung die bzw. jede Schaltstufe durch ein insbesondere periodisches Schaltsignal angesteuert wird und zur Regelung der von der HF-Energiequelle abgegebenen Spannung die von der Gleichspannungsquelle abgegebene Spannung geregelt wird. Auf diese Weise ist eine besonders einfache und kostengünstige Ausgestaltung einer erfindungsgemäß ausgebildeten Vorrichtung möglich. Das Schaltsignal besteht dabei üblicherweise aus Rechteckpulsen, wobei die Frequenz des Schaltsignals typischerweise im Bereich von 300 bis 1000 kHz liegt.

Bei einer weiteren vorteilhaften Ausführungsform der Erfindung wird die von der Gleichspannungsquelle abgegebene Leistung und/oder der von der Gleichspannungsquelle abgegebene Strom erfaßt, so daß die Leistungs-und/oder Stromerfassung auf der Primärseite der HF-Energiequelle erfolgt. Dazu ist das Meßelement zum Erfassen der von der HF-Energiequelle abgegebenen Leistung und/oder des Stroms zwischen der Gleichspannungsquelle und der Schaltstufe oder innerhalb der Gleichspannungsquelle oder der Schaltstufe angeordnet. Bei der Erfassung des Stroms und/oder der Leistung auf der Primärseite der HF-Energiequelle müssen bei der Ermittlung des Stroms und/oder der Leistung Transformationsfaktor und Wirkungsgrad der Schaltstufen sowie eventuell weiterer vorhandener Elemente, wie beispielsweise eines vorhandenen Spannungsreglers berücksichtigt werden.

Durch die Erfassung des Stroms und/oder der Leistung auf der Primärseite der HF-Energiequelle werden zusätzliche Schaltelemente auf der Sekundärseite, die zu unzulässigen Patientenableitströmen und zu HF-Leckströmen führen können, vermieden. Weiterhin ist die Erfassung der Leistung und/oder des Stroms auf der Primärseite mit deutlich geringerem Hardwareaufwand verbunden als auf der Sekundärseite.

Grundsätzlich ist es jedoch auch möglich, daß die Leistung und/oder der Strom zwischen der Schaltstufe und dem Ausgang der HF-Energiequelle, d.h. auf der Sekundärseite der HF-Energiequelle erfaßt wird.

Nach einer weiteren bevorzugten Ausführungsform der Erfindung ist jedem Ausgang der HF-Energiequelle eine Schaltstufe und ein Meßelement zugeordnet. Auf diese Weise ist gewährleistet, daß jede Elektrode, die an einem Ausgang angeschlossen ist, individuell und optimal eingestellt werden kann.

Bevorzugt umfaßt die Schaltstufe zumindest einen Transformator, durch den die Primär- und die Sekundärseite der HF-Energiequelle miteinander gekoppelt sind, wobei an der Primärseite zumindest ein insbesondere als Transistor ausgebildeter Schalter vorgesehen ist. Erfolgt die Strom- und/oder Leistungserfassung auf der Primärseite der HF-Energiequelle und damit des Transformators, so kann in dem Fall, daß die Schaltstufe in einem weiten Betriebsbereich im wesentlichen linear arbeitet, diese Erfassung durch Berechnung mit folgenden Formeln erfolgen:

$$P_{HF} = (P_{DC} - P_{DC0}) \times \eta$$

$$R_L = (R_{DC} - R_{DC0}) \times k,$$

wobei $P_{HF}$ die HF-Ausgangsleistung auf der Sekundärseite, $P_{DC}$ die Versorgungsleistung auf der Primärseite, $P_{DC0}$ die Versorgungsleistung ohne HF-Abgabe, $\eta$ der Wirkungsgrad, $R_L$ der Lastwiderstand auf der Sekundärseite, $R_{DC}$ der feststellbare Gleichstromwiderstand auf der Primärseite, $R_{DC0}$ der Gleichstromwiderstand-Offset und k ein schaltungsspezifischer Widerstandstransformationsfaktor ist.

Grundsätzlich ist es jedoch auch möglich, daß die Bestimmung der Sekundärwerte aus den Primärwerten über Tabellen oder eine Kombination aus Berechnungen und Tabellen erfolgt. Anstelle von Transistoren können auch beliebige andere Schalter, beispielsweise Röhren, verwendet werden, die der nötigen Schaltfrequenz des Schaltsignals von beispielsweise 300 bis 1000 kHz folgen können.

Nach einer weiteren vorteilhaften Ausführungsform der Erfindung liefern die Gleichspannungsquellen im wesentlichen den gleichen maximalen Spannungswert, wobei insbesondere alle Ausgänge von einer einheitlichen Gleichspannungsquelle gespeist werden. Die Verwendung eines einheitlichen maximalen Spannungswertes für alle Ausgänge bewirkt folgenden erfindungsgemäßen Vorteil. Entsteht aufgrund eines hohen Potentialunterschieds zwischen zwei Katheterelektroden ein hoher Ausgleichsstrom, so wird, wie bereits beschrieben, durch die erfindungsgemäße Regelung des von dem Ausgleichsstrom betroffenen eingeprägten Ausgangsstrom die von der zugehörigen Gleichspannungsquelle abgegebene Spannung solange erhöht, bis die durch den Ausgleichsstrom bewirkte Abweichung vom Sollwert aufgehoben ist. Im Extremfall geht dabei die Schaltung in die Sättigung, da die Gleichspannungsquelle mit ihrem maximalen Wert den Aussteuerbereich begrenzt. Besitzen alle Gleichspannungsquellen den gleichen maximalen Spannungswert, so liefern im Extremfall die den beiden betroffenen Elektroden zugeordneten Spannungsquellen die gleiche Spannung, so daß aufgrund des gleichen Potentials kein Ausgleichsstrom fließen kann.

Vorteilhaft werden die vorgegebenen Leistungs- und/oder Stromwerte durch eine Steuerung vorgegeben, die insbesondere als Temperaturregelung ausgelegt sein kann. Dazu sind im Bereich der Ausgänge der HF-Energiequelle, insbesondere im Bereich der Elektroden temperaturempfindliche Sensoren vorgesehen, die mit der Steuerschaltung verbunden sind. Abhängig von den von den Sensoren gemessenen Temperaturwerten werden durch die Steuerschaltung für jedes Regelelement die gewünschten Leistungs- und/oder Stromwerte bestimmt, die an das Regelelement angelegt werden. Es ist grundsätzlich auch möglich, geeignete andere Meßgrößen zur Regelung zu verwenden. Beispielsweise kann anstelle oder in Ergänzung der Temperaturregelung eine Regelung nach dem absoluten Impedanzwert und/oder nach Änderungen des Impedanzwertes eingesetzt werden.

Weitere bevorzugte Ausführungsformen sind in den Unteransprüchen angegeben.

Die Erfindung wird nachfolgend anhand von Ausführungsbeispielen unter Bezugnahme auf die Zeichnungen näher beschrieben; in diesen zeigen:

Figur 1 ein Blockschaltbild einer Ausführungsform einer erfindungsgemäß ausgebildeten Vorrichtung,

Figur 2 eine schematische Darstellung einer Eintakt-Schaltstufe mit einer regelbaren Gleichspannungsquelle und einem Stromflußüberwachungselement, wie sie bei einer Vorrichtung gemäß Figur 1 verwendbar ist,

Figur 3 eine schematische Darstellung einer Gegentaktschaltstufe mit regelbarer Spannungsquelle und Stromflußüberwachungselement,

Figur 4 eine schematische Darstellung einer Halbbrücken-Schaltstufe mit regelbarer Spannungsquelle und Stromflußüberwachungselement und

Figur 5 eine schematische Darstellung einer Vollbrücken-Schaltstufe mit regelbarer Spannungsquelle und einem Stromflußüberwachungselement.

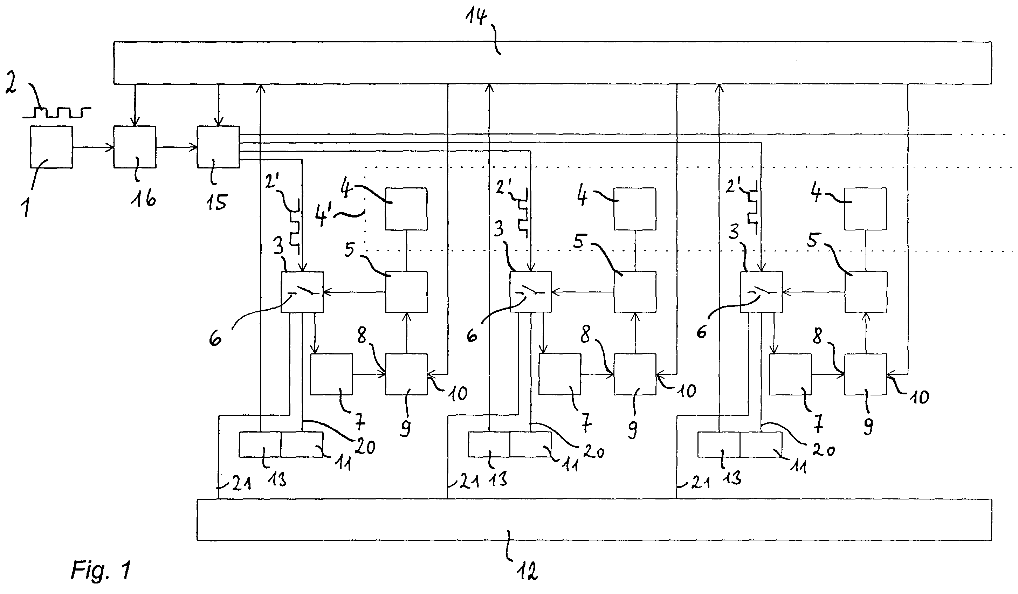

Die in Figur 1 dargestellte Vorrichtung umfaßt einen Taktgeber 1, der ein periodisches, rechteckförmiges Schaltsignal 2 für eine Vielzahl von Schaltstufen 3 erzeugt.

Jeder Schaltstufe 3 ist eine Gleichspannungsquelle 4 zugeordnet, die die Schaltstufe 3 jeweils über einen Spannungsregler 5 mit einer variablen Spannung versorgt. Die Gleichspannungsquellen 4 liefern dabei jeweils den gleichen Spannungswert, so daß anstelle unterschiedlicher Spannungsquellen 4 auch eine einheitliche Spannungsquelle 4' verwendet werden kann, wie dies in Figur 1 gestrichelt dargestellt ist.

Die Schaltstufen 3 enthalten jeweils mindestens einen schematisch angedeuteten, beispielsweise als Transistor oder als Röhre ausgebildeten Schalter 6, der durch das an der jeweiligen Schaltstufe 3 anliegende Schaltsignal 2' angesteuert, d.h. geöffnet und geschlossen wird. Durch das Ansteuern des Schalters 6 wird die von dem Spannungsregler 5 an den Schalter 6 angelegte Gleichspannung zerhackt, wodurch die HF-Ausgangsspannung der Schaltstufe 3 erzeugt wird. Somit wird durch die von dem Spannungsregler 5 an die Schaltstufe 3 gelieferte Spannung die Ausgangsspannung der jeweiligen Schaltstufe 3 bestimmt.

Der über den Schalter 6 fließende Strom bzw. die entsprechende Leistung wird für jede Schaltstufe 3 jeweils von einem Meßelement 7 erfaßt und einem Istwerteingang 8 eines Regelelements 9 zugeführt. Das Meßelement 7 kann dabei beispielsweise auch in dem Pfad zwischen dem Spannungsregler 5 und der Schaltstufe 3 oder in dem Pfad zwischen der Gleichspannungsquelle 4 und dem Spannungsregler 5 angeordnet sein. An diesen Stellen wird somit der Strom bzw. die Leistung auf der Primärseite der Schaltstufe 3 gemessen, wodurch das Auftreten von zusätzlichen Ableitströmen durch zusätzliche Schaltungselemente auf der Sekundärseite vermieden wird.

Das Regelelement 9 stellt den von dem Meßelement 7 erfaßten Strom bzw. die erfaßte Leistung entsprechend einem an einem Sollwerteingang 10 des Regelelements anliegenden Sollwert ein und steuert den Spannungsregler 5 so an, daß die Versorgungsspannung für die jeweilige Schaltstufe 3 so geregelt wird, daß der erfaßte Strom bzw. die erfaßte Leistung konstant gehalten wird.

Auf den Sekundärseiten der Schaltstufen 3 sind jeweils Ausgänge 20, 21 der HF-Energiequelle ausgebildet, die zum einen jeweils mit einer Katheterelektrode 11 und zum anderen mit einer gemeinsamen indifferenten Elektrode 12 verbunden sind.

Im Bereich jeder Elektrode 11 ist jeweils ein temperaturempfindlicher Sensor 13 angeordnet, der zur Abgabe eines von der gemessenen Temperatur abhängigen Ausgangssignals mit einer Steuerschaltung 14 verbunden ist. Die Steuerschaltung 14 wiederum liefert abhängig von der gemessenen Temperatur sowie eventuell einer voreingestellten Solltemperatur den Sollwert für die Regelelemente 10.

Während bei der bisher beschriebenen Schaltung die an den Schaltstufen 3 anliegenden Schaltsignale 2' jeweils in Phase sind, ist es durch einen dem Taktgeber 1 nachgeschalteten Phasenschieber 15 möglich, die Phasenlage der Schaltsignale 2' gezielt gegeneinander zu verschieben, wodurch definierte Ausgleichsströme zwischen den Elektroden 8 erzeugt werden können. Diese Ausgleichsströme fließen an der Oberfläche des Gewebes und können dazu dienen, Läsionslücken zwischen den Katheterelektroden 11 zu schließen.

Zwischen dem Taktgeber 1 und dem Phasenschieber 15 ist weiterhin eine Unterbrecherschaltung 16 angeordnet, mit der die von dem Taktgeber 1 erzeugten Schaltsignale 2 regelmäßig unterbrochen werden können, um ein Pulsen der Ausgangsleistung zu erzeugen. Dadurch kann vorteilhaft das Läsionsprofil gesteuert werden, wobei bevorzugt sowohl die Dauer als auch die Frequenz der durch die Unterbrecherschaltung 16 erzeugten Unterbrechungen einstellbar sind.

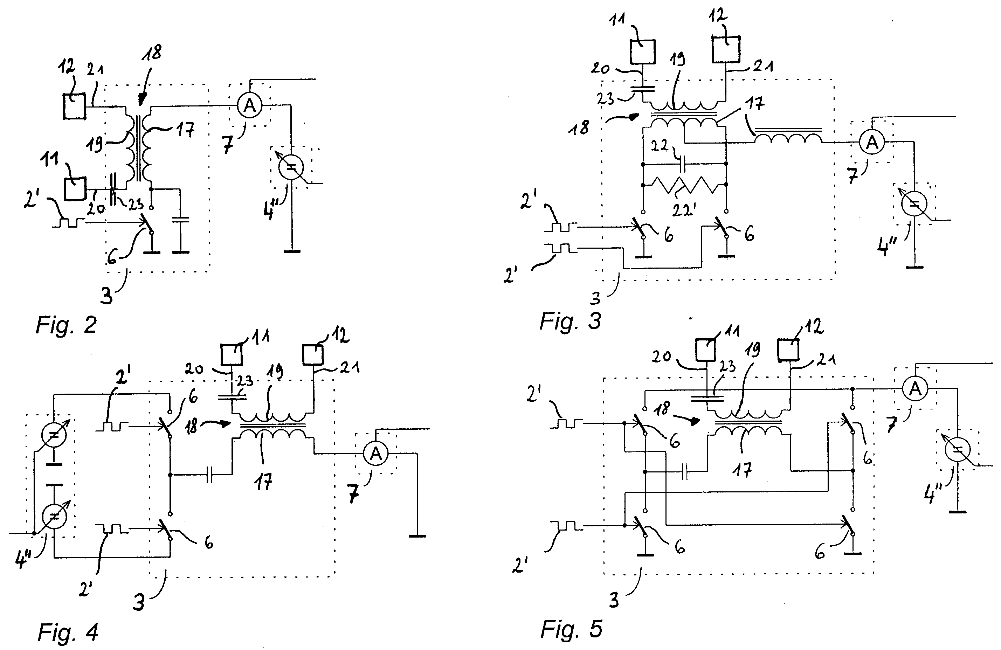

Figur 2 zeigt eine Ausbildung der Schaltstufe 3 als Eintakt-Schaltstufe, wobei zum besseren Verständnis zusätzlich ein Teil der in Figur 1 bereits dargestellten, mit der Schaltstufe 3 verschalteten Bauelemente ebenfalls in Figur 2 dargestellt sind.

Der Schalter 6 der Schaltstufe 3 ist in Reihe mit einer Primärwicklung 17 eines Transformators 18 geschaltet, wobei das andere Ende der Primärwicklung 17 mit dem Meßelement 7 verbunden ist. Die Sekundärwicklung 19 des Transformators 18 ist mit ihrem einen Anschluß üblicherweise über einen Kondensator 23 mit der Katheterelektrode 11 und mit dem anderen Anschluß mit der indifferenten Elektrode 12 verbunden.

Die in Figur 1 dargestellte Gleichspannungsquelle 4 sowie der Spannungsregler 5 sind in den Figuren 2 bis 5 jeweils zu einer geregelten Spannungsquelle 4'' zusammengefaßt.

Wie der Figur 2 zu entnehmen ist, wird durch die Ansteuerung des Schalters 6 durch das Schaltsignal 2' die von der Gleichspannungsquelle 4'' gelieferte Spannung zerhackt und über den Transformator 18 von der Primärwicklung 17 auf die Sekundärwicklung 19 übertragen. Der dabei induzierte Strom fließt über die Katheterelektrode 11 in das Gewebe zur indifferenten Elektrode 12, wodurch im Bereich der Katheterelektrode 11 das Gewebe koaguliert wird.

Während die in Figur 2 dargestellte Eintakt-Schaltstufe lediglich durch ein einziges Schaltsignal 2' angesteuert wird, werden die in den Figuren 3 bis 5 dargestellten Schaltstufen jeweils durch zwei gegenphasige Schaltsignale 2' angesteuert. Wie bei der Eintakt-Schaltstufe nach Figur 2 ist auch bei den Schaltstufen gemäß den Figuren 3 bis 5 jeweils die Sekundärwicklung 19 des Transformators 18 mit der Katheterelektrode 11 und der indifferenten Elektrode 12 verbunden, so daß mit diesen Schaltstufen der gleiche Effekt erzielt werden kann, wie mit der Eintakt-Schaltstufe gemäß Figur 2.

Die Funktionsweise einer erfindungsgemäß ausgebildeten Vorrichtung wird anhand der Figuren nachfolgend nochmals näher beschrieben:

Durch den Taktgeber 1 wird ein Schaltsignal 2 erzeugt, das durch die Unterbrecherschaltung 16 zeitweise unterbrechbar und durch den Phasenschieber 15 in Schaltsignale 2' mit unterschiedlicher Phasenlage umsetzbar ist.

Die Schaltsignale 2' steuern jeweils den/die Schalter 6 der Schaltstufe 3 an, so daß die von der Gleichspannung 4 gelieferte und über den Spannungsregler 5 eingestellte, an der Schaltstufe 3 anliegende Gleichspannung in eine Wechselspannung umgesetzt wird. Der primärseitig fließende Strom wird durch das Meßelement 7 gemessen und an das Regelelement 9 weitergegeben, das aufgrund eines von der Steuerschaltung 14 anliegenden Sollwertes für Strom oder Leistung den Spannungsregler 5 so ansteuert, daß der erfaßte Strom oder die Leistung konstant bleibt.

Sekundärseitig ist die Schaltstufe 3 über die Ausgänge 20, 21 der HF-Energiequelle mit der Katheterelektrode 11 sowie der indifferenten Elektrode 12 verbunden, so daß das an der Katheterelektrode 11 anliegende Gewebe aufgrund der an dieser Stelle auftretenden hohen Stromdichte erhitzt und denaturiert wird.

Die im Bereich der Katheterelektrode 11 herrschende Temperatur wird durch den Sensor 13 gemessen und an die Steuerschaltung 14 übertragen, durch die ein entsprechender Sollwert an das Regelelement 9 angelegt wird.

Besteht zwischen zwei nebeneinanderliegenden Katheterelektroden 11 ein Potentialgefälle, so ist es möglich, daß ein Ausgleichsstrom von der Elektrode 11 mit der höheren Spannung zu der Elektrode 11 mit der geringeren Spannung fließt. Da sich dieser Ausgleichsstrom mit dem eingeprägten Ausgangsstrom der Elektrode 11 mit der niedrigeren Spannung überlagert, steuert nach Erfassen des resultierenden Stromwertes durch das Meßelement 7 das Regelelement 9 den Spannungsregler 5 so an, daß die an der Schaltstufe 3 anliegende Spannung nachgeregelt wird, so daß der Sollwert des Stroms oder der Leistung wieder eingestellt wird.

Im Extremfall wird dabei die an der Schaltstufe 3 anliegende Spannung bis zur Sättigungsgrenze geregelt, wobei durch die Verwendung einer einheitlichen Spannungsquelle 4' gewährleistet ist, daß in diesem Extremfall sowohl an der den Ausgleichsstrom abgebenden Elektrode 11 als auch an der den Ausgleichsstrom aufnehmenden Elektrode 11 die gleiche Spannung anliegt, so daß zwischen diesen beiden Elektroden 11 kein Ausgleichsstrom fließen kann.

Während bisher beschrieben wurde, daß jeder Katheterelektrode ein Temperatursensor zugewiesen ist, ist es grundsätzlich auch möglich, daß für jede Elektrode mehrere Sensoren, für mehrere Elektroden ein gemeinsamer Referenzsensor oder an einem Teil der Elektroden bzw. an allen Elektroden kein Sensor vorgesehen wird.

Die Verwendung der beschriebenen Schaltstufen ist von Vorteil, da diese einen hohen Wirkungsgrad aufweisen, da die Verlustleistungen über die Schalter gering sind.

Um die Leistungsrückspeisung weiter verringern zu können, kann es möglich sein, alle Schalter in die Offenstellung zu schalten, falls die entsprechende Schaltstufe mit einer Versorgungsspannung von 0 Volt angesteuert werden soll.

Unerwünschte Ausgleichsströme können in einer vorteilhaften Weiterbildung auch dadurch verhindert werden, daß keine Schaltungselemente eingesetzt werden, die eine signifikante passive Stromeinprägung erlauben würden, wie dies beispielsweise bei Entlastungsnetzwerken der Fall ist. Weiterhin werden bevorzugt Schaltungselemente vorgesehen, die den Oberwellengehalt der Ausgangsspannung verringern. Durch Oberwellen kann die Funktion von in der Nähe betriebenen Geräten gestört werden, so daß durch entsprechende Schaltungselemente, beispielsweise parallel zur Primärwicklung des Ausgangstransformators geschaltete Kondensatoren 22 (Fig. 3) oder eine Kondensator-Widerstandskombination 22, 22' (Fig. 3), die Amplituden der Oberwellen vermindert werden können.

Grundsätzlich ist es auch möglich, daß anstelle einer gemeinsamen indifferenten Elektrode weitere Elektroden beispielsweise auf dem gleichen oder einem anderen Katheter vorgesehen sind, die als Gegenpol zu den beschriebenen Katheterelektroden dienen. Darüber hinaus kann die Schaltung modular ausgebildet sein, so daß beispielsweise die Ansteuerschaltung jeder einzelnen Elektrode durch ein separates Modul gebildet sein kann.

Es kann insbesondere für medizinische Anwendung auch sinnvoll sein, die HF-Energiequelle mit einer spannungsgesteuerten Charakteristik zu versehen. Daher kann es sinnvoll sein, die Regelelemente sowohl mit einer Strom- als auch mit einer Spannungsregelung zu versehen, wobei vorteilhaft durch die Lastverhältnisse bestimmt wird, welche der beiden Regelungen wirksam ist. Die für die Spannungsregelung benötigte Messung des Spannungswertes kann wiederum wahlweise entweder auf der Primär- oder auf der Sekundärseite der Schaltstufen erfolgen.

## Bezugszeichenliste

| | |
|---|---|
| 1 | Taktgeber |
| 2, 2' | Schaltsignal |
| 3 | Schaltstufen |
| 4, 4', 4" | Gleichspannungsquelle |
| 5 | Spannungsregler |
| 6 | Schalter |
| 7 | Meßelement |
| 8 | Istwerteingang |
| 9 | Regelelement |
| 10 | Sollwerteingang |
| 11 | Katheterelektrode |
| 12 | indifferente Elektrode |
| 13 | Sensor |
| 14 | Steuerschaltung |
| 15 | Phasenschieber |
| 16 | Unterbrecherschaltung |
| 17 | Primärwicklung |
| 18 | Transformator |
| 19 | Sekundärwicklung |
| 20 | Ausgang |
| 21 | Ausgang |
| 22 | Kondensator |
| 22' | Widerstand |
| 23 | Kondensator |

## Patentansprüche

1. Verfahren zum Betrieb einer Hochfrequenz-Ablationsvorrichtung, mit einer mehrere regelbare Ausgänge (20) aufweisenden HF-Energiequelle, an deren Ausgänge (20) eine Vielzahl von Elektroden (11) anschließbar sind,
   **dadurch gekennzeichnet,**

   daß für jeden Ausgang (20) jeweils ein für die von der HF-Energiequelle abgegebene Leistung und/oder den Strom repräsentativer Leistungs- und/oder Stromwert erfaßt wird, daß die von der HF-Energiequelle abgegebene Spannung in Abhängigkeit von dem erfaßten Leistungs- und/oder Stromwert so geregelt wird, daß dieser im wesentlichen einem vorgegebenen Leistungs- und/oder Stromwert entspricht und daß zwischen den Strömen oder zwischen den Spannungen an den Ausgängen (20) der HF-Energiequelle jeweils eine vorgegebene Phasenbeziehung eingehalten wird.

2. Verfahren nach Anspruch 1,
   **dadurch gekennzeichnet,**

   daß die HF-Energiequelle zumindest eine regelbare Gleichspannungsquelle (4, 4', 5, 4") und zumindest eine mit der Gleichspannungsquelle (4, 4', 5, 4") verbundene Schaltstufe (3) umfaßt, daß zur Erzeugung der HF-Ausgangsspannung die bzw. jede Schaltstufe (3) durch ein insbesondere periodisches Schaltsignal (2, 2') angesteuert wird, daß zur Regelung der von der HF-Energiequelle abgegebenen Spannung die von der Gleichspannungsquelle (4, 4', 5, 4") abgegebene Spannung geregelt wird und insbesondere, daß die von der Gleichspannungsquelle (4, 4', 5, 4") abgegebene Leistung und/oder der von der Gleichspannungsquelle (4, 4', 5, 4") abgegebene Strom erfaßt wird, d. h. die Leistungs- und/oder Stromerfassung auf der Primärseite der HF-Energiequelle erfolgt, oder daß die Leistung und/oder der Strom zwischen der Schaltstufe (3) und dem Ausgang (20) der HF-Energiequelle, d.h. auf der Sekundärseite der HF-Energiequelle erfaßt wird.

3. Verfahren nach Anspruch 2,
   **dadurch gekennzeichnet,**

   daß jedem Ausgang (20) der HF-Energiequelle eine Schaltstufe (3) zugeordnet ist und daß zumindest ein Teil der Schaltstufen (3) durch phasensynchrone, insbesondere durch phasengleiche Schaltsignale (2), oder durch gegeneinander phasenverschobene Schaltsignale (2') angesteuert werden, wobei bei der Verwendung von phasenverschobenen Schaltsignalen (2') die Phasenverschiebung insbesondere einstellbar ist.

4. Verfahren nach einem der Ansprüche 2 oder 3,
   **dadurch gekennzeichnet,**

   daß die Schaltsignale (2, 2') in insbesondere regelmäßigen Abständen für unterschiedlich lange oder gleich lange Zeitabschnitte unterbrochen werden, insbesondere daß die Zeitpunkte und/oder die Frequenz und/oder die Dauer der Unterbrechungen einstellbar sind.

5. Verfahren nach einem der Ansprüche 2 bis 4,
   **dadurch gekennzeichnet,**

   daß die Gleichspannungsquellen (4, 4', 4") im wesentlichen den gleichen maximalen Spannungswert liefern, insbesondere daß eine einheitliche Gleichspannungsquelle (4') für alle Ausgänge (20) verwendet wird.

6. Verfahren nach einem der vorhergehenden Ansprüche,
   **dadurch gekennzeichnet,**

   daß im Bereich von an den Ausgängen (20) angeschlossenen Elektroden (11) Temperaturwerte und/oder absolute Impedanzwerte und/oder Impedanzänderungen gemessen werden und daß die vorgegebenen Leistungs- und/oder Stromwerte in Abhängigkeit von den gemessenen Werten bestimmt werden.

7. Vorrichtung für die Hochfrequenz-Gewebe-Ablation, mit einer mehrere regelbare Ausgänge (20) aufweisenden HF-Energiequelle, an deren Ausgänge (20) eine Vielzahl von Elektroden (11) anschließbar sind,
   **gekennzeichnet durch**

   zumindest ein Meßelement (7) zur Erfassung von für die von der HF-Energiequelle an den Ausgängen jeweils abgegebene Leistung und/oder den Strom repräsentativen Leistungs- und/oder Stromwerten und zumindest ein mit dem Meßelement (7) verbundenes Regelelement (9) zur Regelung der von der HF-Energiequelle abgegebenen Spannung in Abhängigkeit von dem an einem Istwerteingang (8) des Regelelements (9) anliegenden erfaßten Leistungs- und/oder Stromwert und einem an einem Sollwerteingang (10) des Regelelements (9) anliegenden vorgegebenen Leistungs- und/oder Stromwert, wobei die Ströme oder Spannungen an den Ausgängen (20) der HF-Energiequelle jeweils eine vorgegebene Phasenbeziehung zueinander besitzen.

8. Vorrichtung nach Anspruch 7,
   **dadurch gekennzeichnet,**

   daß die HF-Energiequelle zumindest eine regelbare Gleichspannungsquelle (4, 4', 5, 4"), zumindest eine mit der Gleichspannungsquelle (4, 4', 5, 4") verbundene Schaltstufe (3) und einen ein insbesondere periodisches Schaltsignal (2) abgebenden Taktgeber (1) umfaßt, durch den die Schaltstufe (3) ansteuerbar ist.

9. Vorrichtung nach Anspruch 8,
   **dadurch gekennzeichnet,**

   daß das Meßelement (7) zum Erfassen der von der HF-Energiequelle abgegebenen Leistung und/oder des Stroms zwischen der Gleichspannungsquelle (4, 4', 5, 4") und der Schaltstufe (3) oder innerhalb der Gleichspannungsquelle (4") oder der Schaltstufe (3), d. h. auf der Primärseite der HF-Energiequelle, angeordnet ist oder daß das Meßelement (7) zum Erfassen der von der HF-Energiequelle abgegebenen Leistung und/oder des Stroms zwischen der Schaltstufe (3) und dem Ausgang (20) der HF-Energiequelle, d. h.

auf der Sekundärseite der HF-Energiequelle, angeordnet ist.

10. Vorrichtung nach einem der Ansprüche 8 oder 9, **dadurch gekennzeichnet,**

daß jedem Ausgang (20) der HF-Energiequelle eine Schaltstufe (3) und ein Meßelement (7) zugeordnet sind und/oder daß die Schaltstufe (3) zumindest einen Transformator (18) umfaßt, durch den die Primär- und die Sekundärseite der HF-Energiequelle miteinander gekoppelt sind, wobei an der Primärseite zumindest ein insbesondere als Transistor oder Röhre ausgebildeter Schalter (6) vorgesehen ist.

11. Vorrichtung nach einem der Ansprüche 8 bis 10, **dadurch gekennzeichnet,**

daß ein Phasenschieber (15) vorgesehen ist, mit dem die Phasenlage der die Schaltstufen (3) ansteuernden Schaltsignale (2'), insbesondere wahlweise einstellbar, gegeneinander verschiebbar ist und/oder
daß ein Unterbrechungselement (16) zur insbesondere wahlweise einstellbaren Unterbrechung der Schaltsignale (2, 2') vorgesehen ist und/oder daß die Gleichspannungsquellen (4, 4', 4") im wesentlichen den gleichen maximalen Spannungswert liefern, insbesondere daß alle Ausgänge (20) von einer einheitlichen Gleichspannungsquelle (4') gespeist werden und/oder daß die Schaltstufen (3) als Eintakt-Schaltstufen, als Gegentakt-Schaltstufen, als Halbbrücken-Schaltstufen und/oder als Vollbrücken-Schaltstufen ausgebildet sind.

12. Vorrichtung nach einem der Ansprüche 7 bis 11, **dadurch gekennzeichnet,**

daß im Bereich von an den Ausgängen (20) angeschlossenen Elektroden (11) temperaturempfindliche Sensoren (13) und/oder Impedanzmeßelemente vorgesehen sind, daß die Sensoren (13) bzw. die Impedanzmeßelemente mit einer Steuerschaltung (14) zur Bestimmung der vorgegebenen Leistungs- und/oder Stromwerte abhängig von den gemessenen Temperaturwerten bzw. von der gemessenen absoluten Impedanz oder von gemessenen Impedanzänderungen verbunden sind und daß die Steuerschaltung (14) mit dem Regelelement (9) verbunden ist.

13. Hochfrequenz-Ablationskatheter mit einer Vorrichtung nach einem der Ansprüche 7 bis 12 und mit einer Vielzahl von Elektroden (11), die jeweils mit den Ausgängen (20) der HF-Energiequelle verbunden sind.

14. Katheter nach Anspruch 13, **dadurch gekennzeichnet,**

daß jeweils genau eine Elektrode (11) mit genau einem Ausgang (20) verbunden ist.

Fig. 1

EP 0 925 761 A1

9

Fig. 3

Fig. 5

Fig. 2

Fig. 4

## EP 0 925 761 A1

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| X | US 5 693 078 A (DESAI) 2. Dezember 1997 <br> * Zusammenfassung; Abbildungen 3A,4,5,12 * <br> * Spalte 6, Zeile 13-26 * | 1,6 | A61B17/39 |
| Y | * Spalte 9, Zeile 64 - Spalte 10, Zeile 13 * | 2,5 | |
| A | US 5 630 837 A (CROWLEY) 20. Mai 1997 | 1 | |
| Y | WO 96 39086 A (VALLEYLAB) 12. Dezember 1996 | 2,5 | |
| A | | 8,10 | |
| A | bezugszeichen 16, 17 <br> * Abbildung 1 * <br> * Seite 66, Zeile 25-33 * <br> * Seite 3, Zeile 5 - Zeile 12 * | 3 | |
| X | WO 97 40760 A (CARDIAC CRS MOMINEES) 6. November 1997 <br> * Zusammenfassung * <br> * Seite 15, Zeile 25 - Seite 16, Zeile 11 * <br> * Seite 2, Zeile 6-10 * | 7 | RECHERCHIERTE SACHGEBIETE (Int.Cl.6) <br><br> A61B |
| A | WO 96 39088 A (VALLEYLAB) 12. Dezember 1996 <br> * Seite 5, Zeile 1 - Seite 7, Zeile 17 * | 1,2,7 | |

Der vorliegende Recherchenbericht wurde für alle Patentansprüche erstellt

| Recherchenort | Abschlußdatum der Recherche | Prüfer |
|---|---|---|
| DEN HAAG | 20. April 1999 | Papone, F |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**   EP 98 12 0431

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

20-04-1999

| Im Recherchenbericht angeführtes Patentdokument | Datum der Veröffentlichung | Mitglied(er) der Patentfamilie | Datum der Veröffentlichung |
|---|---|---|---|
| US 5693078 A | 02-12-1997 | US 5383917 A | 24-01-1995 |
| | | US 5620481 A | 15-04-1997 |
| | | AU 663511 B | 12-10-1995 |
| | | AU 2248592 A | 11-02-1993 |
| | | CA 2112821 A | 21-01-1993 |
| | | EP 0598742 A | 01-06-1994 |
| | | EP 0904800 A | 31-03-1999 |
| | | JP 7500025 T | 05-01-1995 |
| | | MX 9203954 A | 01-01-1993 |
| | | WO 9300958 A | 21-01-1993 |
| US 5630837 A | 20-05-1997 | AU 5382296 A | 16-10-1996 |
| | | EP 0764004 A | 26-03-1997 |
| | | JP 10502290 T | 03-03-1998 |
| | | WO 9629935 A | 03-10-1996 |
| | | CA 2165829 A | 19-01-1995 |
| | | EP 0706345 A | 17-04-1996 |
| | | JP 9503677 T | 15-04-1997 |
| | | WO 9501751 A | 19-01-1995 |
| | | US 5840031 A | 24-11-1998 |
| | | US 5588432 A | 31-12-1996 |
| WO 9639086 A | 12-12-1996 | AU 5700796 A | 24-12-1996 |
| WO 9740760 A | 06-11-1997 | AU 2374897 A | 19-11-1997 |
| WO 9639088 A | 12-12-1996 | AU 5700996 A | 24-12-1996 |
| | | CA 2220909 A | 12-12-1996 |
| | | EP 0836433 A | 22-04-1998 |
| | | JP 10506564 T | 30-06-1998 |

EPO FORM P0461

Für nähere Einzelheiten zu diesem Anhang : siehe Amtsblatt des Europäischen Patentamts, Nr. 12/82